# EUROPEAN PATENT APPLICATION

(11) **EP 4 389 112 A1**
(43) Date of publication of application: **26.06.2024**
(21) Application number: 22216579.7
(22) Date of filing: 23.12.2022
(51) Int. Cl.: A61K 9/00, A61K 9/51, A61K 49/00, A61K 49/22, C08J 3/12, C08J 3/24

(54) **CAVITATION-INDUCING BIODEGRADABLE POLYMERIC PARTICLES**

(71) Applicant: Oxsonics Limited, Oxford, OX4 4GA (GB)
(72) Inventor: The designation of the inventor has not yet been filed
(74) Representative: J A Kemp LLP

(57) **Abstract**

The present invention provides a cup-shaped particle comprising a first polymer and a second polymer; wherein the first polymer is a biodegradable polymer; the second polymer is a crosslinked copolymer of two or more different methacrylate monomers and at least one crosslinking agent, wherein the at least one cross-linking agent comprises one or more dimethacrylates; and wherein the cup has a cavity having an opening of at least 50 nm in size.

## Description

### Field of the Invention

The invention relates to cavitation-inducing biodegradable polymeric micro- or nanoparticles.

### Background

Micro- and nanoscale initiators of inertial cavitation are known in the art, and have been shown to be useful in biomedical applications, and non-biomedical applications. Biomedical applications include histotripsy, thermal ablation, targeting and enhancement of drug delivery, sonothrombolysis and diagnostic ultrasound imaging. Non-biomedical applications include sonochemistry, sonofusion, water treatment and the use of ultrasound to characterize material properties, such as those disclosed in WO 2011/036475.

In order to be therapeutically useful, there are many criteria which a cavitation initiator must satisfy. For example, achieving uniform cavitation in a liquid would require an agent that can remain in suspension for extended periods of time, which is not possible with either buoyant microbubbles or sinking microparticles. Further, whilst blood vessels are typically no smaller than 8 microns in diameter, the tight junctions between endothelial cells lining the vessel wall are smaller than 100 nm in healthy tissue and between 100-800 nm in diseased or cancerous tissue. Therefore microbubbles are confined to the blood vessels, and are incapable of extravasating into the perivascular space and subsequent tissue layers. This is particularly limiting in therapeutic applications, such as drug delivery, seeking to treat tissue that is remote from blood vessels. Cavitation initiators of sub-micron size are therefore desirable.

In addition, it is important to provide cavitation initiators which are capable of reliably generating inertial cavitation at clinically relevant levels.

While inertial cavitation agents which meet these criteria have been described, for example in WO 2015/075442, it has been necessary to use plastic materials, such as polystyrenes to form particles with satisfactory cavitation properties. However, such plastic particles accumulate and persist primarily in the liver and spleen and are therefore not suitable for use in long term treatment regimes or in potentially curative treatments, given that the particles remain indefinitely in the body after any treatment is complete.

There is therefore a need for improved inertial cavitation agents, which can satisfy all the requirements met by existing therapeutically viable cavitation agents, but do not persist in the subject.

### Summary of the invention

The present invention provides cup-shaped micro- or nanoarticles which reliably generate inertial cavitation at clinical relevant levels and which are biodegradable. The cup-shaped particles are acoustically tuneable, i.e. the production of the particles can be accurately controlled to produce a cavitation initiator of sub-micron size, which is tuned to respond to unique acoustic settings. Further, the biodegradable nature of the particle means it naturally degrades in a subject, avoiding the problems of long-term accumulation observed with prior articles. Therefore, the particles have the potential to be used for prolonged periods of treatment with reduced risk of negative side-effects.

Thus, the present invention provides a cup-shaped micro- or nanoparticle comprising a first polymer and a second polymer; wherein, the first polymer is a biodegradable polymer; the second polymer is a crosslinked copolymer of two or more different methacrylate monomers and at least one crosslinking agent, wherein the at least one cross-linking agent comprises one or more dimethacrylates; and wherein the cup has a cavity having an opening of at least 50 nm in size. Typically the micro- or nanoparticle is a particle of from 60nm to 1µm in size.

Both the first and second polymers are biodegradable and therefore the particle itself is biodegradable. Therefore, the particles described herein do not persist in a subject for long periods after treatment but rather are broken down within the body.

The invention further provides a method of producing a cup-shaped particle according to the invention, the method comprising providing a seed particle, wherein said seed particle comprises a first polymer which is a biodegradable polymer; and polymerising one or more monomers and/or pre-oligomers and/or pre-polymers and at least one crosslinking agent on the surface of the seed particle to produce a cup having a cavity.

Also provided by the invention is a pharmaceutical composition comprising a plurality of cup-shaped particles as defined anywhere herein, or obtainable according to the method as described anywhere herein, and a pharmaceutically acceptable carrier or diluent which is a liquid medium.

In a further embodiment, the invention provides a solid composition comprising a plurality of cup-shaped particles as defined anywhere herein, or obtainable according to the method as described anywhere herein, the cup-shaped particles being in dried form.

The invention further provides a cup-shaped particle as defined anywhere herein, or obtainable according to the method as described anywhere herein, or a composition as defined anywhere herein, for use in the treatment of a human or animal subject, wherein the method comprises administering the cup-shaped particle, or the composition, to the subject and exposing the subject to ultrasound.

Also provided by the invention is an ultrasound contrast agent, comprising a cup-shaped particle as defined anywhere herein, or obtainable according to the method as described anywhere herein, or a composition as defined anywhere herein.

### Description of the figures

Figure 1 depicts a typical cup-shaped particle according to the invention.
Figure 2 depicts schematically the process for producing cup-shaped particles according to the invention.
Figure 3 depicts TEM and SEM images of the particles prepared in the Examples.
Figure 4 depicts cavitation power of three formulations SGP130, SGP136 and SGP137 vs. water and 5% glucose as a control.
Figure 5 depicts percentage change in the optical density relative to the optical density recorded at t=0 weeks. Data has been corrected for any change in volume occurred throughout the study such as evaporation or the addition of reagents to maintain the required oxidative conditions.
Figure 6 depicts (left) Turbidity of SGP137, PLGA seeds and SGP136 at the start of the study, (right) Turbidity of SGP130, SGP136, SGP137, and SEEDS at the end of the study T6 week.
Figure 7 depicts TEM images of SGP130, SGP136 AND SGP137 at each pull point. Scale bar represents 2 µm.

### Detailed description of the invention

### Cup-shaped particles

The particles of the invention are cup-shaped. As used herein, cup-shaped indicates that the particles have a cavity which is capable of holding a gas bubble. The gas bubble is typically partially encapsulated by the cavity, but may not be fully within the cavity. The terms gas bubble and gas pocket are used interchangeably herein.

Where a gas pocket is present in the cup-shaped particle, the particle comprises:
- a cup having a cavity, wherein the cavity having an opening of at least 50 nm in size, and
- a gas pocket present in the cavity,
wherein the gas pocket is partially encapsulated by the cup.

The ability to stabilise a gas bubble within the cavity of the cup-shaped particle provides the ability to generate inertial cavitation on exposure to ultrasound. For example and without wishing to be bound by theory, the cup-shaped particles of the invention may generate inertial cavitation when a gas bubble contained in the cavity expands on exposure to ultrasound of suitable frequency and pressure. Inertial cavitation occurs when the bubble rapidly collapses, causing mechanical streaming effects in the surrounding fluid. These mechanical streaming effects may physically propel the particles and any co-administered agents such as a drug or diagnostic agent. These effects can therefore be used to drive the particle and co-administered agent to a desired target site such as a tumour.

The particles of the invention are microparticles or nanoparticles, preferably microparticles. As used herein, a microparticle is a particle having a size of from 0.1 to 10 µm. Preferred microparticles have a size of up to 1 µm, e.g. from 0.1 to 1 µm. As used herein, a nanoparticle is a particle having a size of from 1 to 100 nm. Preferred nanoparticles have a size of at least 60nm, e.g. from 60 to 100nm. Therefore, the microparticles and nanoparticles described herein are typically particles having a size of from 60nm to 1 µm, preferably 100 to 1000nm.

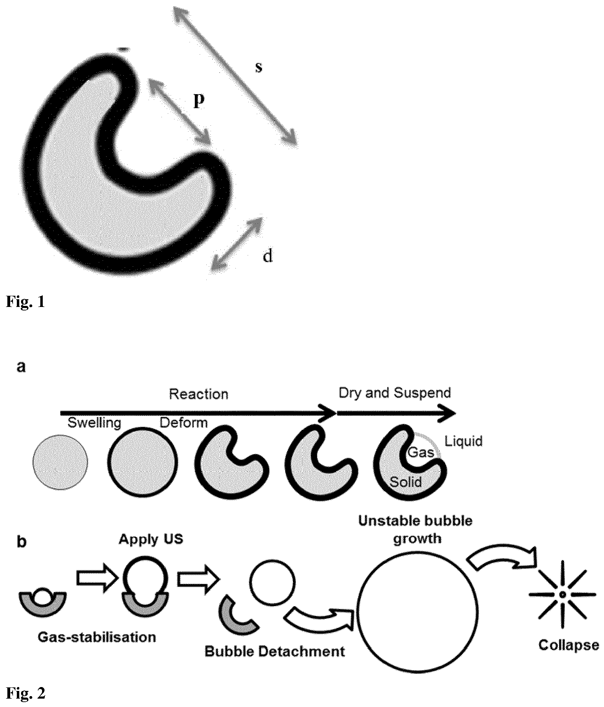

The size of the particle is typically the mean diameter of the particle (s in Figure 1). The diameter is typically determined by electron microscopy in a dried state. Where a plurality of cup-shaped particles are present in a composition, typically as a suspension, the cumulant average is typically taken as the size of the particles. This can be determined by dynamic light scattering.

As used herein the size of the opening of a cavity is the maximum dimension of the cavity measured at the rim of the cup (p in Figure 1), typically the part of the cup that forms a boundary between an encapsulated part of the gas pocket and an un-encapsulated part of the gas pocket. In the case of a substantially part-spherical cavity, the opening will be substantially circular and the size is measured as the diameter of the opening. The size of the opening of a cavity can be determined by transmission electron microscopy.

As used herein, the depth of the cup refers to the maximum distance from the level of the rim of the cup to the base of the cup (d in Figure 1). The depth of the cup can be determined by transmission electron microscopy.

As used herein the term "part-spherical" describes a shape that forms part of a sphere. The term "part-spherical" includes shapes such as a spherical cap, a hemisphere, and a sphere having its top truncated above its equator (i.e. the part of a sphere complementary to a spherical cap). Substantially part-spherical includes shapes such as those forming parts of a spheroid, and parts of a sphere or spheroid having irregularities such as depressions and protrusions.

Biodegradable, as used herein, refers to any material which breaks down in the body naturally. Typically, a biodegradable polymer, or a biodegradable particle is broken down into smaller constituent parts by biological processes, for example degradation by enzymes in the body. Without wishing to be bound by theory, the particles described herein contain ester bonds, so are thought to be broken down by esterase enzymes in the body, e.g. hydrolase. The biodegradable particles and biodegradable polymers may also be broken down by oxidative enzymes, such as peroxidase, catalase and xanthine oxidase that can accelerate the degradation rate *in-vivo.* These enzymes may play an important role in the degradation by catalysing the hydrolysis of the polymers.

Typically, a biodegradable polymer or biodegradable particle is substantially cleared from the body within a period of less than 18 months, and typically less than 12 months. The ability for a particle to degrade *in vivo* may be assessed using *in vitro* accelerated degradation studies. In one such technique, particles are maintained under oxidative conditions of 3% H₂O₂, pH 4.5-5.0 and at a temperature of 69 ± 1 °C in an incubator shaker at 200 rpm. Degradation is typically assessed by measuring optical density. A biodegradable particle typically has an optical density of less than 10%, preferably less than 5%, preferably substantially zero, within a period of 12 weeks, preferably 8 weeks, more preferably 6 weeks when subject to these accelerated degradation conditions. A biodegradable polymer typically is a polymer which, when formed into a particle having a size of about 500nm, has an optical density of substantially zero within a period of 12 weeks, preferably 8 weeks, more preferably 6 weeks when subject to these accelerated degradation conditions.

The cup-shaped particle of the present invention comprises a first polymer and second polymer. The cup-shaped particle also has a cavity having an opening of at least 50 nm in size.

The first polymer used herein may be any a biodegradable polymer. The biodegradable polymer is preferably hydrophobic. The biodegradable polymer is preferably insoluble in water at standard temperature and pressure (i.e. 25 °C and 100,000 Pa), wherein insoluble, as used herein, means that the polymer has a solubility in water of less than 1 part per 10,000 parts water (by mass) at standard temperature and pressure (i.e. 25 °C and 100,000 Pa). The hydrophobicity of the polymer is beneficial in the emulsion seed polymerisation process which is used to form the cup-shaped particles. In particular, a hydrophobic polymer is able to form seeds in water. These hydrophobic seeds attract the hydrophobic monomers of the second polymer, to their surface, such that the second polymer forms on the surface of the seed. This therefore reduces or eliminates polymerisation in the medium of the polymerization reaction and maximises polymerisation on the seed surface.

Examples of suitable polymers for use as the first polymer include polyesters and polysaccharides, in particular poly(lactic-co-glycolic acid) (PLGA), polyglycolic acid (PGA), poly(lactic acid) (PLA), poly(dioxanone), poly(ethylene succinate), poly (butylene succinate), chitosan, and poly(hydroxyalkanoate) (PHA). The PHA should preferably have a melting point above 120°C.

In some embodiments, the first polymer is preferably PLA or PLGA, most preferably PLGA. When the first polymer is PLGA the blend of lactic acid to glycolic acid is typically from 25:75 to 80:20 weight percent, preferably from 40:60 to 60:40 weight percent. In some embodiments, the blend is from 45:55 to 55:45.

The second polymer used herein is a crosslinked copolymer of two or more different methacrylate monomers, and at least one crosslinking agent, wherein the at least one crosslinking agent comprises one or more dimethacrylates, typically the second polymer is a copolymer of two or more different methacrylate monomers and one or more dimethacrylate cross-linkers.

The nature of the second polymer is important in providing the biodegradable properties of the particles. In particular, previous particles used cross-linking agents such as divinyl benzene. Where the shell of a particle is formed of divinyl benzene and other monomers, the second polymer has a rigid structure which is beneficial for providing a particle which can withstand the forces which the particle will be subject to during inertial cavitation and prevent shattering. The particles also reliably cavitate at a pre-determined ultrasound frequency and pressure. However, the particles cannot break down in the body. The present inventors have determined that by use of dimethacrylates, in particular the dimethacrylates described herein, as cross-linking agents, it is possible to provide particles that have the beneficial properties imparted by use of divinyl benzene, namely rigidity and strength of structure to enable inertial cavitation to occur, and repeatability of production of particles of a constant shape such that the particle is acoustically tunable. However, the use of dimethacrylates as cross-linking agents also allows the particles to biodegrade, because the presence of two ester groups, in the main structure of the cross-linker, makes the cross-linker hydrolysable.

Therefore, the second polymerused herein, which makes the shell of the particle, is a biodegradable polymer. This biodegradable polymer is preferably hydrophobic. The hydrophobicity of the particle shell ensures that it is suitable to hold a gas bubble in its cavity.

The two or more different methacrylate monomers which are used to form the second polymer are typically alkyl or hydroxyalkyl methacrylates, typically wherein the alkyl and/or the hydroxyalkyl are from one to six carbons in length, preferably one to four carbons in length, preferably one to two carbons in length. Straight chain alkyl or hydroxyalkyl groups are typically used. The hydroxyalkyl group is typically an alkyl group having one hydroxy substituent. In some preferred embodiments the two or more different methacrylate monomers are methyl methacrylate (MMA) and (hydroxyethyl)methacrylate (HEMA).

In the second polymer, the typical weight percentage ratio of methacrylates to crosslinking agent is (methacrylate:crosslinking agent) from 30:70 to 70:30, preferably from 40:60 to 60:40.

When the two or more different methacrylate monomers are methyl methacrylate (MMA) and (hydroxyethyl)methacrylate (HEMA), the second polymer typically comprises MMA, crosslinking agent and HEMA in a weight percentage ratio of (30-55):(40-60):(5-10).

At least one crosslinking agent is used to form the second polymer. Typically one, two or three different cross-linking agents are used, more typically one or two different cross-linking agents, for example one crosslinking agent. The at least one crosslinking agent comprises one or more dimethacrylates, typically one or two different dimethacrylates. The or each dimethacrylate is typically an alkylene glycol dimethacrylate of formula (I): 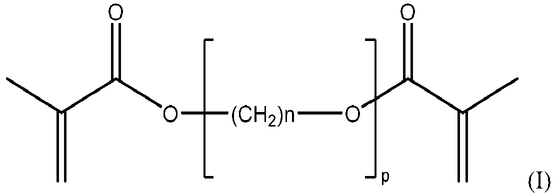 wherein n is 2 or 3, preferably 2; and
p is 1, 2 or 3, preferably 1 or 2, most preferably 1.

In formula (I), preferably n is 2 and the dimethacrylate is therefore an ethylene glycol dimethacrylate having one, two or three ethylene glycol groups. Most preferably, n is 2 and p is 1 or 2.

Preferably, therefore, the one or more dimethacrylates are selected from ethylene glycol dimethacrylate (EGDMA) and di(ethylene glycol) dimethacrylate (DEGDMA) and combinations thereof. In some preferred embodiments, the crosslinking agent is EGDMA. Particles wherein the cross-linking agent is EGDMA may show improved biodegradability.

When the crosslinking agent comprises EGDMA or a combination of EGDMA and DEGDMA, the weight percentage ratio of EGDMA : DEGDMA may be from 100:0 to 25:75. Typically the ratio is from 100:0 to 50:50.

Degree of monomer conversion may be determined by various chromatographic methods, such as high-performance liquid chromatography (HPLC).

Therefore in some preferred embodiments the second polymer is a crosslinked polymer of MMA, HEMA and one or more dimethacrylates, typically MMA, HEMA and one or two dimethacrylates. Preferably, the second polymer is a crosslinked polymer of MMA, HEMA and one or more dimethacrylates selected from EGDMA and DEGDMA and combinations thereof. Most preferably, the second polymer is a crosslinked polymer of MMA, HEMA and EGDMA.

In some preferred embodiments, the weight percentage ratio MMA:dimethacrylate:HEMA is (30-55):(40-60):(5-10). In some embodiments, the weight percentage ratio MMA:EGDMA:HEMA is (30-55):(40-60):(5-10).

The cup-shaped particle may comprise the first polymer, as described anywhere herein, and the second polymer, as described anywhere herein, in a weight percentage ratio (first polymer:second polymer) of from 25:75 to 45:55.

The first and second polymer as described herein, have been surprisingly found to form cup-shaped particles which reliably cavitate at clinically relevant levels and are biodegradable. Therefore, the present invention provides a cup-shaped particle which is biodegradable. Thus, the particles of the present invention have the potential to be used in long-term treatment of subjects, with reduced risk of adverse side-effects resulting from long-term accumulation of the particles in a subject, for example in the liver of the subject.

The length of time that it takes for a particle of the invention to fully degrade in a physiological environment can be tuned by altering the composition (the weight percentage ratio MMA:dimethacrylate, e.g. EGDMA:HEMA) of the particle, as needed. Typically, a particle of the invention will fully degrade in less than 18 months, and typically less than 12 months.

The cup-shaped particle is typically biocompatible, i.e. capable of performing its desired function with respect to a medical therapy or method of diagnosis without eliciting any therapeutically unacceptable local or systemic effects in the recipient or beneficiary of that therapy or diagnosis.

The particles typically have a size of at least 60 nm or at least 100 nm, preferably at least 200nm, at least 300nm or at least 400nm, and up to 1000 nm, e.g. up to 800nm or up to 600 nm. For example, the particle may have a size of from 60 to 1000 nm or from 200 to 800nm, or from 300 to 600 nm, preferably from 400 to 800nm, from 400 to 600nm or from 400 to 500 nm.

In embodiments where the particle is for use in a method of treating a tumour, then a particular size of the particle may be desired in order to improve accumulation in tumour tissue by the enhanced permeability and retention (EPR) effect. Tumour tissues may contain neovasculature having abnormal form and architecture, leading to abnormal molecular and fluid transport dynamics. That can cause particles of around 100 to 500 nm, e.g. 100 to 300 nm in size to accumulate in tumour tissue much more than they do in normal tissues. Particle sizes of 100 to 500 nm, e.g. 400 to 500 nm may therefore be desired, in particular for use in methods of treating a tumour.

The cup-shaped particles are typically substantially part-spherical. For example the particles may be part-spherical.

At least part of the cavity of the cup-shaped particle, optionally the entire cavity, is typically substantially part-spherical, e.g. substantially hemispherical or hemispherical. The cavity has an opening size (p) of at least 50 nm. The cavity typically has an opening size (p), (i.e. the diameter at the opening when the cavity is part-spherical), of from 50 to 900 nm, e.g. at least 50 nm, at least 100 nm or at least 200 nm. Typically the opening size (p) is no more than 700 nm, 600 nm, 500 nm, no more than 400 nm or no more than 300nm. The opening size of the cavity may, for example, be from 50 to 300 nm, from 50 to 400 nm, from 50 nm to 500 nm, or from 100 to 500 nm or from 100 to 600 nm. The opening size of the cavity is preferably from 50 nm to 500 nm. A skilled person will be able to select an appropriate cavity shape and size to complement desired ultrasound parameters, and produce particles having the desired cavity shapes and sizes based on the methods disclosed herein for producing the particles.

The ratio of the size of the opening to the size of the particle (ratio p:s in Figure 1) is typically from 1:3 to 5:6, for example from 1:2 to 2:3.

The cavity typically has a depth of more than 30nm, preferably more than 50nm. For example, the cavity typically has a depth of from 60 to 500nm, for example from 80 to 400nm.

Cavities having a larger size (e.g. a depth of greater than 30nm, preferably greater than 50nm or more; and/or an opening of 50nm or more) have the advantage that greater cavitation effects can be generated.

Each cup-shaped particle may comprise a single gas pocket in its cavity. Thus the invention provides a cup-shaped particle which is a single cup having a single cavity. The use of particles having a single cup or cavity, and a single gas pocket stabilised therein, provides a more predictable and reliable response to applied ultrasound. By control of the size of the cup-shaped particle, and the size of the stabilised gas bubble, the particle can be tuned to respond to defined ultrasound conditions. Shallow or small indentations or imperfections on the surface of a particle are not considered to be cavities, in the context of the present invention. Such small indentations or imperfections typically cannot stabilise a gas pocket in their cavity. Thus, the opening of the cavity is at least 50nm in size. Typically, any indentation having a depth of less than about 5nm, typically less than about 10nm or less than about 20nm is not considered a cavity in the context of the present invention. Therefore, a particle having a single cavity may comprise one or more such indentations having an opening size of less than 50nm and/or having a depth of less than about 5nm, typically less than about 10nm or less than about 20nm, on its surface.

When a gas pocket is present in the cavity, the gas pocket may be partially encapsulated by the cup. A gas pocket which is partially encapsulated by the cup is typically a gas pocket having a part of its surface which is encapsulated by the cup, optionally with one or more layers present between the cup and the gas pocket, and a part of its surface which is not encapsulated by the cup and may be exposed to a medium in which the particle may be present, e.g., a liquid medium. This enables the expansion and collapse of the gas pocket which leads to inertial cavitation.

The gas pocket is typically a bubble having a diameter of at least 50 nm. For example, the gas pocket may have a diameter of from 50 to 900 nm, e.g. at least 50 nm, 100 nm or at least 200 nm. The gas pocket may have a diameter of no more than 700 nm, 600 nm, 500 nm or no more than 400 nm. The gas pocket may, for example, have a diameter of from 50 to 400 nm, from 50 nm to 500 nm, or from 100 to 600 nm. In some preferred embodiments, the gas pocket has a diameter of from 50 nm to 500 nm.

The desired size and shape of the cavity containing the gas pocket is determined by the ultrasound frequency to be used, typically in the range 100 kHz to 5000 kHz, in order to minimize the rarefactional pressure amplitude required to initiate inertial cavitation. Typically, the ultrasound frequency to be used is at least 100 kHz, such as at least 200 kHz, or at least 400 kHz. The ultrasound frequency may be up to 1000 kHz, such as up to 800 kHz, or up to 600 kHz.

Ultrasound pressure amplitudes less than 5 MPa are preferred, e.g. 3MPa or less, 2.5MPa or less, or 2MPa or less. Ultrasound pressure amplitudes may be at least 0.5 MPa, or at least 1 MPa. Ultrasound pressure amplitudes as low as 0.5 MPa can yield extensive inertial cavitation at frequencies as high as 2000 kHz in the presence of the cup-shaped particles.

When the cup-shaped particle comprises a gas pocket, the gas present in the gas pocket is not particularly limited but is typically air, nitrogen, oxygen, a perfluorocarbon such as perfluoropropane, or a mixture thereof.

The gas pocket may be the same size and shape as the cavity. For example, if the cavity is a hemisphere with a diameter d at its opening, then the gas pocket may be a hemisphere of diameter d. Alternatively, the gas pocket may be larger than the cavity, i.e. it may protrude from the rim of the cup. It may protrude from the rim of the cup a distance that is 10% or more of the depth of the cup, e.g. 20% or more, 50% or more 80% or more or 100% or more. The depth of the cup and the degree to which the gas pocket may protrude can be determined using transmission electron microscopy. In some embodiments the cavity is part-spherical or substantially part-spherical with a curvature of radius *r*, and the gas pocket is a sphere with radius *r.* In an example of this embodiment the cavity is a hemisphere with a diameter *d* at its opening, and the gas pocket is a sphere of radius ½ *d*.

For the cup-shaped particle of the invention the cavity size may be controlled in order to provide tuneable cavitation initiators. The cup-shaped particles of the invention therefore typically have a cavity opening size which is predictable and repeatable. Such cup-shaped particles having predictable cavity opening size are therefore tuneable cup-shaped particles.

### Cup-shaped particle compositions

The particles may be provided as a composition e.g. a pharmaceutical composition. This may be either a composition comprising a plurality of particles alone in dried form (e.g. as a powder), or a composition comprising the particles together with a pharmaceutically acceptable carrier or diluent. Typically, compositions provided together with a carrier or diluent are provided as liquid compositions comprising a suspension of particles in a liquid medium. This facilitates delivery of the particles, which is typically by parenteral administration. Particles may optionally be stored in dried form and resuspended prior to use. Said pharmaceutical composition typically contains up to 85 wt% of a particle of the invention. More typically, it contains up to 50 wt% of a particle of the invention. Preferred pharmaceutical compositions are sterile and pyrogen free.

Compositions comprising a plurality of particles in dried form are typically resuspended in a suitable liquid, e.g. an aqueous solution prior to use.

Suitable pharmaceutically acceptable carriers and diluents include those suitable for parenteral administration, in particular aqueous solutions and sterile water. Glucose, saccharose, mannitol, glycerine, sorbitol or combinations thereof may be present in the aqueous solution. Dilute glucose solutions, e.g. 0.5-10%, preferably 1-8%, e.g. about 5% glucose solution may be used. If desired, the pharmaceutical composition may comprise one or more further excipients, carriers, buffers, stabilisers, antioxidants or other materials, as required. The pharmaceutically acceptable carriers, diluents and other excipients, buffers, stabilisers, antioxidants or other materials should be non-toxic and should not interfere with the efficacy of the particles.

Compositions comprising a plurality of particles typically comprise particles which are substantially uniform. This has the advantage that greater control over cavitation is achieved and thus improved acoustic tuning. The preferred polydispersity index of the composition of particles is 0.2. This is typically determined by dynamic light scattering.

The average particle size of a composition comprising a plurality of particles is defined herein as the modal average (i.e. the particle size displayed by the maximum number of particles). In a preferred aspect of the invention, at least 50% of the particles in the composition have a particle size within 100nm of the average. More preferably at least 60%, 70% or 80% of the particles have a particle size within 100nm of the average. In a further preferred aspect of the invention, at least 50% of the particles in the composition have a particle size within 50nm of the average. More preferably at least 60%, 70% or 80% of the particles have a particle size within 50nm of the average. Yet more preferably, at least 50% of the particles in the composition have a particle size within 20nm of the average. More preferably, at least 60%, 70% or 80% of the particles have a particle size within 20nm of the average. Dynamic light scattering may be used to determine the variation in particle size within a composition.

The concentration of particles in the composition can be determined by the skilled person depending on the subject and the ultrasound parameters which are to be used. Typical compositions contain at least 0.01mg/mL and up to 500 mg/mL. Preferably, the concentration of particles in the composition is in the range of 10 to 100 mg/mL.

The average cavity size (the size of the opening of the cavity, p) of a composition comprising a plurality of particles is defined herein as the modal average (i.e. the cavity size displayed by the maximum number of particles). In a preferred aspect of the invention, at least 50% of the particles in the composition have a cavity size within 50nm of the average. More preferably at least 60%, 70% or 80% of the particles have a cavity size within 50nm of the average. Yet more preferably, at least 50% of the particles in the composition have a cavity size within 20nm of the average. More preferably, at least 60%, 70% or 80% of the particles have a cavity size within 20nm of the average. Electron microscopy may be used to determine the variation in cavity opening size within a composition.

### Process for producing cup-shaped particles

The particles of the invention can be produced by a method comprising providing a seed particle, wherein said seed particle comprises a first polymer which is a biodegradable polymer; and polymerising one or more monomers and/or pre-oligomers and/or pre-polymers and at least one crosslinking agent on the surface of the seed particle to produce a cup having a cavity.

The seed particle is formed of the first polymer as described anywhere herein. The one or more monomers and/or pre-oligomers and/or pre-polymers and the cross-linking agents form the second polymer as described anywhere herein. Thus, the cross-linking agent is one or more cross-linking agents as described anywhere herein. The one or more monomers and/or pre-oligomers and/or pre-polymers are two or more different methacrylate monomers as described anywhere herein and/or pre-oligomers or pre-polymers thereof. Optionally pre-oligomers or pre-polymers used in the polymerisation step may be pre-oligomers or pre-polymers of one or more methacrylates as described anywhere herein formed with a cross-linking agent such as a dimethacrylate cross-linking agent.

The seed particle may be coated (for example fully coated, or partially coated, for example 50% or more, typically 60%, 70%, 80%, 90%, 95% or more of the surface area of the seed particle is coated) by the step of polymerising one or more monomers and/or pre-oligomers and/or pre-polymers and at least one crosslinking agent on the surface of the seed particle to produce a cup having a cavity.

Optionally the method further comprises a step of drying the cup. In particular, the method may comprises a further step of providing gas to the cup to produce a pocket of gas within the cavity.

The particles can be produced by forming a cup by a seeded emulsion polymerisation technique, optionally forming a drug layer in the cavity of the cup, and drying the cup in the presence of a gas to introduce the gas pocket.

The size and shape of the cavity can be selected and accurately controlled by selecting the size of the seed particles to be used. Thus, the seeded emulsion polymerisation technique typically uses a seed particle having a size and shape complementary with the desired size and shape of the cavity. For example, a spherical seed particle can be used to produce a cup having a part-spherical cavity. Using larger seed particles can form a larger cavity, and similarly, using smaller seed particles can produce a smaller cavity. Seed particles used will typically have a size of 50 to 900 nm, e.g. at least 50nm or at least 100nm and up to 600nm, up to 400nm or up to 300nm.

Emulsion polymerisation is typically carried out by mixing seed particles, monomers, and a cross linking agent in a suitable medium such as water, mixing to produce turbulent conditions and adding a suitable initiator such as potassium persulfate. Thus the method may comprise initiating the polymerisation reaction using an initiator. The emulsion polymerisation reaction is allowed to continue at a suitable temperature for a suitable period of time. A skilled person will be able to select appropriate temperature and time parameters for the particular reaction mixture used, but the polymerisation typically takes place over 1-6 hours at a temperature of 70 to 90 °C, e.g. for about 1.5 hours at about 80 °C.

As the cup-shaped particle is formed from two or more monomer units, the different monomers may be provided to the emulsion polymerisation at the beginning of polymerisation, or alternatively one or more monomers may be provided part way through the polymerisation process. Alternatively, one or more of the monomer units may be pre-polymerised and a pre-oligomer and/or a pre-polymer provided to the polymerisation mixture, either at the beginning of polymerisation or part way through polymerisation. Typically, one or more of the monomers,pre-oligomers or pre-polymers may have a greater affinity for the seed particle. Thus, the polymerisation may provide a cup-shaped particle having one monomer unit predominantly formed on the inner surface of the cup-shaped particle (i.e. the monomer having greatest affinity for the seed particle) and a different monomer unit predominantly on the exterior surface of the cup-shaped particle. This may provide a cup-shaped particle having differing surface properties on the interior and exterior surfaces.

The seed particle remains incorporated within the interior of the cup-shaped particle, the seed particle material typically being itself incorporated into the core of the cup-shaped particle. Typically, the particles therefore comprise a core which is formed of a blend of the first polymer (derived from the seed particle) and the second polymer. The shell, or surface of the cup-shaped particle is typically formed of the second polymer. For example, the shell or surface of the particle may comprise at least 90%, e.g. at least 95% or at least 98% of the second polymer. There is typically no distinct boundary between the core and shell of the particle, rather a gradual increase in the amount of first polymer which is blended with the second polymer moving from the surface or shell of the particle to its core.

In the case of a complete reaction of all available seed particles, there will typically be no seed particles remaining that need to be removed. Large agglomerates and small by-products are removed through centrifugation and filtration. The final shape, size and successful formation of the cup-shaped particle can be verified by transmission electron microscopy (TEM) acquired at multiple tilt angles of the grid plane, that can be reconstructed into a 3D model. Preferably, the level of by-products in the cup-shaped particle is minimised. By-products can be minimised by controlling the stirring rate of the polymerisation reaction and by washing them out through centrifugation. By-products typically make up no more than 5% by weight, e.g. 2% or 1% by weight of the cup-shaped particle.

After emulsion polymerisation has been carried out, the product can be washed, e.g., by centrifuging, in order to remove any excess monomers. After washing the product is typically suspended in a suitable medium such as water.

The product may then be dried by any suitable technique such as freeze-drying or air-drying. If freeze-dried a cryoprotectant is not typically used. Air-drying is preferred and may be carried out at elevated temperatures, for example, or in a desiccator. Air-drying may be carried out over a period of 12-36 hours, e.g., 24 hours. In one embodiment, air drying is carried out at a temperature of 40 to 50 °C for 6 to 18 hours, e.g., about 12 hours and then in a desiccator for 14 hours at 18-24 °C.

If dried in air the gas pocket in the particles of the invention will be air gas pockets. Alternative gasses can be used for the gas pocket by carrying out the drying step in the desired gas. For example, drying can be carried out in air, and the resulting particles having an air gas pocket can be subjected to a vacuum to remove the air gas pocket, and contacted with the desired gas to introduce a new gas pocket of the desired gas. Drying the cup-shaped particles in a gaseous atmosphere provides the required gas pocket within the cavity of the cup. Prior to drying, no gas pocket is present.

If dried, the cup-shaped particles may be resuspended in a liquid medium such as an aqueous solution. Suitable solutions include dilute glucose solution, e.g. 0.5-10%, preferably 1-8%, e.g. about 5% glucose solution. Typically, the solution is then mixed to maximise particle dispersion. Dried cup-shaped particles retain their gas pocket on resuspension. Thus, cup-shaped particles which have been dried in a gaseous atmosphere and resuspended differ from those formed directly in solution, in that a gas pocket is present in the cavity, due to the drying step.

Following resuspension of the cup-shaped particles, the solution is preferably filtered to remove agglomerated material. Syringe filtration, for example at 1-5µm, typically 1.5-3µm, may be used to remove agglomerated particles. This limits the particles present to those having a particle size of less than the filtration limit. A combination of coarse filtration followed by syringe filtration may be used.

Where cup-shaped particles in dried form are desired, for example for ease of transportation, the resuspension and filtration steps can be omitted. Dried cup-shaped particles are typically resuspended and, depending on the resulting size distribution, the suspension optionally filtered, prior to use.

When the cup-shaped particle comprises one or more release agents, the method may further comprise providing one or more release agents (a) as a coating on the seed particle; and/or (b) during polymerisation; and/or (c) to the exterior surface of the cup. Often, two or more different release agents are provided.

Figure 2 schematically depicts the process of producing the cup-shaped particles according to the invention. Fig 2a) shows individual template particles which were coated with a co-polymer, which induced swelling and deformation of the spherical particle. The resulting cup-shaped particle was dried and suspended to entrap gas in the cavity. Fig 2b) gives a diagrammatic representation of the proposed mechanism for inertial cavitation for particle systems. Initially the bubble is stabilized within the cavity. With enough negative pressure, the bubble can extend onto the mouth of the cavity, and eventually detach from the particle. For inertial cavitation to occur, the detached bubble must grow unstably before finally collapsing under the inertia of the surrounding medium.

### Cup-shaped particles comprising release agents

The cup-shaped particles of the invention may be used as drug delivery vehicles by incorporating a release agent, typically a drug, for example either within the cavity of the cup, within the cup, or on the exterior surface of the cup. Therefore, a cup-shaped particle of the invention may further comprise one or more release agents. The release agent may form a layer which is typically on the exterior surface of the cup, in the cup or on the interior surface of the cup, within the cavity. A release agent as referred to herein is a material that is released from the particle after administration, for example the release agent may be released gradually as the particle degrades, or it may be released on cavitation. Typically, the release agent is a drug, i.e. a therapeutic or diagnostic, for example a pharmaceutical or biological material. Thus, references herein to a drug include both small molecule drugs and biologic drugs such as antibodies and other protein-based drugs. Typically, the release agent is a material that is useful in therapy or diagnosis of a human or animal subject. The description herein refers to a drug or a drug layer being present in and/or on the surface of the particle. However, it should be understood that alternative materials, which are usefully released on cavitation, for example when used in non-medical applications, may also be used as the release agent. Any reference to a drug or drug layer therefore is also envisaged as being replaced by an alternative release agent or release agent layer where appropriate.

In embodiments where a drug layer is present in the cavity or within the cup-shaped particle, the nature of the drug is not particularly limited and any drug that may be useful in a method of therapy may be used. Hydrophilic drugs are preferred. Certain drugs include, but are not limited to, anticancer drugs such as doxorubicin, paclitaxel, cisplatin, gemcitabine, daunorubicin, and oxiloplatin, thrombolytic agents such as recombinant tissue plasminogen activator (rt-PA), anti-inflammatories such as acetylsalicylic acid, ibuprofen, salsalate, or antibiotic agents such as geldanamycin or penicillin. Where drugs are incorporated on the exterior of the cup, the drug is typically selected so that it can be bound to the cup in a manner which avoids dissolution on administration. Thus, the drug is released only on cavitation.

The particle may comprise two or more different drugs. For example, one drug may be provided within the cavity with a separate, different, drug being provided on the exterior surface of the cup. Thus, in some embodiments, the invention provides a particle comprising a first release agent in the cavity of the cup, and a second release agent on an exterior surface of the cup. The two drugs may be combined on release, for example on exposure to ultrasound. Thus, the particles may be used as a means to provide a combination therapy, or to provide simultaneous release of two drugs that mix only at the target site when released from the particle vehicle, e.g. following ultrasound exposure.

The addition of a drug layer can occur before formation of the cup (e.g., in the seed particle), during formation of the cup, or after formation of the cup. The timing of the incorporation of the drug layer will result in its placement on the cup-shaped particle. For example, drug laden seed particles will place the drug in the cavity of the cup. Addition of a drug during polymerisation will place the drug within the cup. The addition of drug after formation of the cup will place the drug on an exterior surface of the cup (i.e. a surface which does not face the gas pocket).

The thickness of the drug layer is dependent on the amount and location of the drug. Where the drug is incorporated into the cup itself, the thickness of the drug layer will be constrained by the wall thickness of the cup. In the case of a drug present within the cavity, the drug layer should be less than the depth of the cavity, thus enabling a gas pocket to be present at least partially encapsulated by the cavity. Controlling the thickness of the drug layer can be accomplished by modulating the duration of polymerisation and quantity of drug reactant. Drug loading is measured using drug release profiles.

### Alternative Particles

The invention as described above relates particularly to cup-shaped particles. However, it is envisaged that particles formed of the combination of materials herein may be useful in therapeutic and diagnostic applications in the form of different types of particle. Accordingly, also provided herein is a particle comprising a first polymer and a second polymer; wherein the first polymer is a biodegradable polymer; and the second polymer is a crosslinked copolymer of two or more different methacrylate monomers and at least one crosslinking agent, wherein the at least one cross-linking agent comprises one or more dimethacrylates. The nature of the first polymer and the second polymer, and the ratio of first polymer to second polymer, are as described anywhere herein.

The particles of this embodiment are typically formed by seed emulsion polymerisation using a method as described herein, but varying the shape of the seed particle to provide alternative shapes of particle product.

Preferably, the particles have a core which comprises a blend of the first polymer and the second polymer. Preferably, the surface or shell of the particle is formed substantially of the second polymer. Formed substantially of the second polymer as used herein means that the surface or shell of the particle comprises at least 90% by weight, preferably at least 95% by weight or at least 98% by weight of second polymer. For example the surface of the particle may be formed of the second polymer. The amount of the first polymer may gradually increase when moving from the surface or shell of the particle to the core.

Particles of this embodiment may comprise one or more release agents as described anywhere herein.

### Use of Particles

The particles of the invention can be used alone in any situation in which inertial cavitation is desired such as histotripsy, thermal ablation, sonochemistry, sonofusion and waste water treatment. For example, they can be for use in methods that involve using inertial cavitation to cause localised tissue ablation and/or removal in order to achieve a therapeutic outcome. Such methods include for example the treatment of tumours and removal of cardiac tissue in treatment of heart disease.

Therefore, the present invention provides a cup-shaped particle as described anywhere herein or obtainable according to the methods as described anywhere herein, or a composition as described anywhere herein, for use in a method of treatment or diagnosis of a human or animal subject, wherein the method comprises administering the cup-shaped particle, or the composition, to the subject and exposing the subject to ultrasound.

Thus the present invention also provide a method of treating or diagnosing a disease or disorder in a human or animal subject wherein said method comprises administering a cup-shaped particle as described anywhere herein, or obtainable according to the methods as described anywhere herein, or a composition as described anywhere herein, to the subject and exposing the subject to ultrasound.

The present invention also therefore comprises use of a cup-shaped particle as described anywhere herein, or obtainable according to the methods as described anywhere herein, or a composition as described anywhere herein in the manufacture of a medicament for use in a method of treatment or diagnosis of a human or animal subject, wherein the method comprises administering the cup-shaped particle, or the composition, to the subject and exposing the subject to ultrasound.

The particles of the invention can alternatively be used in combination with other agents, such as a drug or a diagnostic agent (e.g. an MRI contrast agent such as a Gd-based MRI contrast agent, or a fluoroscopy contrast agent). The drug may be a small molecule drug or a biologic drug. The particles of the invention can be used to deliver other agents to a target *in vivo* either by administering the particle and the other agent concomitantly or, in the case of a particle comprising a drug, e.g. a drug layer in the cavity of the cup, by using the particle as part of the drug formulation itself.

In the case of a separate agent being used, the particles of the invention may be administered in a single composition with the separate agent, or as a separate composition. Where two compositions are used, these may be administered simultaneously or separately. Simultaneous or substantially simultaneous administration is preferred.

Application of ultrasound following administration of the particle and separate agent causes cavitation to occur. This enhances transport of the agent into cells. A particular advantage of using cavitation to enhance transport of an active agent into cells in this way is that ultrasound can be applied at selected locations, leading to the active agent being transported into cells only in those selected locations.

In one aspect of the invention, the separate agent is encapsulated in a sonosensitive liposome. Such a sonosensitive liposome can release the agent in the presence of inertial cavitation. The particles of the present invention may therefore be administered concurrently with the liposome and ultrasound applied in order to cause cavitation at the desired location, and correspondingly cause release of the active agent at the desired location.

In an alternative aspect of the invention, as discussed above, the active agent may be incorporated into the particle itself. In this case, cavitation may act to both release the agent from the particle as well as aiding transport of the active agent into cells.

Thus, the method as described above may further comprise administering a drug to the subject, wherein exposure to ultrasound causes delivery of the drug to a target site. The target site is typically a diseased tissue in the body. For example the target site may be a tumour, a fibrous tissue (such as a fibrous liver tissue associated with cirrhotic liver disease, such as is may occur in subjects with non-alcoholic fatty liver disease (NAFLD)), an arterial stenosis or a thrombus.

Thus the cup-shaped particles or compositions of the invention may be for use in a method of sonothrombolysis. The cup-shaped particles or compositions of the invention may alternatively be for use in a method of treating tumour. The cup-shaped particles or compositions of the invention may be for use in methods of histotripsy or thermal ablation. In particular, such methods may be methods of tissue ablation and/or removal. Such methods include for example the treatment of tumours, e.g. to remove tumour tissue; or removal of cardiac tissue in treatment of heart disease, e.g. treatment of thrombus.

When the cup-shaped particles comprise one or more release agents (drug), the or each release agent may be released on exposure to ultrasound. When the cup-shaped particle comprises a first and second release agent, these may mix upon exposure to ultrasound.

The particles of the invention may be administered by any suitable route, depending on the nature of the method of treatment, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, intratumoural etc.), transdermally (e.g. by application of a patch, gel or implant) or by inhalation (as a dry powder, a solution, a dispersion, etc). Preferably, the particles are administered by injection.

Following administration of the particles, ultrasound is applied to generate inertial cavitation at the desired site. Ultrasound pressure amplitudes less than 5 MPa are preferred, e.g. 4Mpa or less, or 3MPa or less. Thus, typical peak negative ultrasound pressure is in the range of from 0.5MPa to 5MPa, preferably from 0.5MPa to 4MPa e.g. from 1 to 4MPa, e.g. from 1.5 to 3 MPa. The ultrasound frequency (fc) may be up to 5000 kHz, e.g. up to 2000 kHz, e.g. up to 1000 kHz or about 500 kHz. Typically frequencies used (fc) are in the range of 100 to 5000 kHz, e.g. from 100 to 1000 kHz, or from 200 kHz to 800 kHz. The pulse repetition frequency may be up to 20 kHz, for example up to 1kHz or up to 1Hz. Typically the pulse repetition frequency is up to 10Hz, e.g. up to 1Hz, for example from 0.1 to 1Hz. Ultrasound exposure may continue for up to 120 minutes, e.g. up to 60 minutes, or up to 30 minutes. Typically the ultrasound exposure is carried out at a time when there will be effective circulation of the particle composition in the subject's bloodstream. Preferably, the ultrasound administration is carried out during the period of peak circulation of the composition.

When used in combination with other agents the particles can also provide real time tracking and mapping of drug distribution *in vivo,* for example using conventional ultrasound imaging or passive acoustic mapping of inertial cavitation (WO 2010 052494). Passive acoustic mapping may be used to maintain the cavitation energy within a desired range, for example below 1nJ, or within the range 0.1 pJ to 1 nJ, for example from 0.5 pJ to 0.5 nJ. Variation of the peak negative pressure ultrasound can be used to control the cavitation energy. Passive acoustic mapping may also be used to monitor in real time the delivery of the composition of the invention to a target site, such as a tumour. By monitoring cavitation in the subject, the extent and location of conjugate delivery can be determined.

The invention also provides an ultrasound contrast agent comprising a cup-shaped particle as defined anywhere herein, or obtainable by a method as described anywhere herein; or comprising a composition as defined anywhere herein. The ultrasound contrast agent may further comprises a further contrast agent, such as an MRI contrast agent, a fluorescent contrast agent, or a radiolabelled contrast agent. When the ultrasound contrast agent comprises a further contrast agent this may be incorporated into the particle or may be separate.

The invention also provides an ultrasound contrast agent for use in a method of diagnosis, wherein said method comprises administering the contrast agent to the subject and exposing the subject to ultrasound.

An amount of drug-containing particle to be administered as part of a method of treatment or diagnosis will depend on, for example, the identity and amount of the drug present in the particles. Thus, the dose of the drug-containing particle of the invention will typically be equivalent to or less than the dose of the drug present in the particle if administered alone, i.e. the amount of drug present in the particles will typically be the same or less than the amount that would be administered if in free form. The dose of the drug-containing particle of the invention may be less than the equivalent amount of free drug for example to compensate for the enhanced pharmacokinetics seen in the agents of the invention as described above, for example 95% or less, 85% or less, 80% or less, 75% or less, 70% or less, 65% or less, 60% or less, 55% or less, or 50% or less.

When used alone the amount of the particle administered can be any appropriate dose as can be determined by a skilled person.

The present invention can be further described by the below paragraphs:
[1] A cup-shaped particle comprising a first polymer and a second polymer;
   wherein the first polymer is a biodegradable polymer;
   the second polymer is a crosslinked copolymer of two or more different methacrylate monomers and at least one crosslinking agent, wherein the at least one cross-linking agent comprises one or more dimethacrylates; and
   wherein the cup has a cavity having an opening of at least 50 nm in size.
[2] A cup-shaped particle according to [1], wherein the first polymer is selected from poly(lactic-co-glycolic acid) (PLGA), polyglycolic acid (PGA), poly(L-lactic acid) (PLA), poly(dioxanone), poly(ethylene succinate), poly (butylene succinate), chitosan, and poly(hydroxyalkanoate).
[3] A cup-shaped particle according to [1] or [2], wherein the first polymer is poly(lactic-co-glycolic acid) (PLGA) or poly(L-lactic acid) (PLA).
[4] A cup-shaped particle according to [3], wherein the first polymer is PLGA which comprises a blend of lactic acid to glycolic acid of from 25:75 to 80:20 weight percent, preferably from 40:60 to 60:40 weight percent.
[5] A cup-shaped particle according to any one of [1] to [4], wherein the two or more different methacrylate monomers are methyl methacrylate (MMA) and (hydroxyethyl)methacrylate (HEMA).
[6] A cup-shaped particle according to [5], wherein the second polymer comprises MMA, crosslinking agent and HEMA in a weight percentage ratio of (30-55):(40-60):(5-10).
[7] A cup-shaped particle according to any one of [1] to [6], wherein the one or more dimethacrylates are selected from ethylene glycol dimethacrylate (EGDMA) and di(ethylene glycol) dimethacrylate (DEGDMA) and combinations thereof.
[8] A cup-shaped particle according to [7], wherein the weight percentage ratio of EGDMA : DEGDMA is from 100:0 to 25:75.
[9] A cup-shaped particle according to [8], wherein the crosslinking agent is at least EGDMA.
[10] A cup-shaped particle according to any one of the preceding paragraphs wherein the weight percentage ratio of the first polymer to the second polymer is (first polymer:second polymer) from 25:75 to 45:55.
[11] A cup-shaped particle according to any one of the preceding paragraphs, wherein the cup-shaped particle is a single cup having a single cavity.
[12] A cup-shaped particle according to any one of the preceding paragraphs, wherein at least a part of the cavity of the particle is substantially part-spherical, preferably wherein the cavity of the particle is substantially part-spherical.
[13] A cup-shaped particle according to any one of the preceding paragraphs wherein the cavity has an opening of from 50 nm to 500 nm.
[14] A cup-shaped particle according to any one of the preceding paragraphs, wherein a gas pocket is present in the cavity.
[15] A cup-shaped particle according to [14], wherein the gas pocket is a bubble having a diameter of from 50 nm to 500 nm.
[16] A cup-shaped particle according to any one of the preceding paragraphs, wherein the particle is from 100 nm to 1000 nm in size, preferably from 150 nm to 600 nm in size.
[17] A cup-shaped particle according to any one of the preceding paragraphs, wherein the particle comprises one or more release agents.
[18] A cup-shaped particle according to [17], wherein a release agent is present within the cup, on an exterior surface of the cup and/or in the cavity of the cup.
[19] A cup-shaped particle according to [18], wherein the particle comprises a first release agent in the cavity of the cup, and a second release agent on an exterior surface of the cup.
[20] A cup-shaped particle according to any one of paragraphs [17] to [19], wherein the or each release agent is a drug.
[21] A cup-shaped particle according to any one of the preceding paragraphs, wherein the cup-shaped particle is biodegradable.
[22] A method of producing a cup-shaped particle according to any one of the preceding paragraphs, the method comprising:
   - providing a seed particle, wherein said seed particle comprises a first polymer, which is a biodegradable polymer; and
   - polymerising one or more monomers and/or pre-oligomers and/or pre-polymers and at least one crosslinking agent on the surface of the seed particle to produce a cup having a cavity.
[23] A method according to [22], wherein the method comprises coating the seed particle, wherein the seed particle is coated by the step of polymerising one or more monomers and/or pre-oligomers and/or pre-polymers and at least one crosslinking agent on the surface of the seed particle to produce a cup having a cavity.
[24] A method according to [22] or [23] wherein the method comprises a further step of providing gas to the cup to produce a pocket of gas within the cavity.
[25] A method according to [24], wherein the step of providing gas to the cup to produce a pocket of gas within the cavity is carried out by drying the cup in air.
[26] A method according to any one of paragraphs [22] to [25], wherein said polymerisation is emulsion polymerisation which is carried out in the presence of the seed particle.
[27] A method according to any one of paragraphs [22] to [26], wherein the method comprises a polymerisation initiation step using an initiator, optionally wherein the initiator is potassium persulfate.
[28] A method according to any one of paragraphs [22] to [27], wherein the seed particle has a diameter of 50nm to 1000 nm, preferably 200 to 700 nm.
[29] A method according to any of paragraphs [22] to [28], wherein the cup-shaped particle comprises one or more release agents, and the method further comprises providing one or more release agents (a) as a coating on the seed particle; and/or (b) during polymerisation; and/or (c) to the exterior surface of the cup.
[30] A method according to [29], wherein two or more different release agents are provided.
[31] A pharmaceutical composition comprising a plurality of cup-shaped particles as defined in any one of paragraphs [1] to [21], or obtainable according to any one of paragraphs [22] to [30], and a pharmaceutically acceptable carrier or diluent which is a liquid medium.
[32] A solid composition comprising a plurality of particles as defined in any one of paragraphs [1] to [21], or obtainable according to any one of paragraphs [22] to [30], the cup-shaped particles being in dried form.
[33] A composition according to paragraph [31] or paragraph [32], wherein the composition comprises particles having a polydispersity index of no more than 0.2.
[34] A cup-shaped particle as defined in any one of paragraphs [1] to [21] or obtainable according to any one of paragraphs [22] to [30], or a composition according to any one of paragraphs [31] to [33], for use in a method of treatment or diagnosis of a human or animal subject, wherein the method comprises administering the cup-shaped particle, or the composition, to the subject and exposing the subject to ultrasound.
[35] A cup-shaped particle or composition for use according to paragraph [34], wherein the ultrasound has a pressure amplitude of from 0.5 MPa to 4 MPa, e.g. from 1 MPa to 4 MPa.
[36] A cup-shaped particle or composition for use according to paragraph [34] or paragraph [35], wherein the ultrasound has a frequency of from 200KHz to 800KHz.
[37] A cup-shaped particle or composition for use according to any one of paragraphs [34] to [36], wherein the method further comprises administering a drug to the subject, and wherein the exposure to ultrasound causes delivery of the drug to a target site.
[38] A cup-shaped particle or composition for use according to paragraph [37], wherein said target site is selected from a tumour, a fibrous tissue, an arterial stenosis, and a thrombus.
[39] A cup-shaped particle or composition for use according to any one of paragraphs [34] to [37], wherein the method is a method of sonothrombolysis.
[40] A cup-shaped particle or composition for use according to any one of paragraphs [34] to [37], wherein method is a method of treating a tumour.
[41] A cup-shaped particle or composition for use according to any one of paragraphs [34] to [40], wherein the cup-shaped particle is defined as in paragraph [17] and wherein the or each drug is released from the cup-shaped particle upon exposure to ultrasound.
[42] A cup-shaped particle or composition for use according to any one of paragraphs [34] to [40], wherein the particle is as defined in paragraph [19] and wherein the first and second release agents mix upon exposure of the particle to ultrasound.
[43] An ultrasound contrast agent comprising a cup-shaped particle as defined in any one of paragraphs [1] to [21] or obtainable according to any one of paragraphs [22] to [30], or a composition according to any one of paragraphs [31] to [33].
[44] An ultrasound contrast agent according to paragraph [43] for use in a method of diagnosis, wherein said method comprises administering the contrast agent to the subject and exposing the subject to ultrasound.

### Examples

### Example 1 - Preparation of PLGA Seed Particles

**Equipment:** Overhead stirrer with 55 mm polytetrafluoroethylene (PTFE) anchor, 5 L beaker/0.5 L Chemglass jacketed reactor, peristaltic pump, microfluidic manifold equipped with 2x or 8x PTFE capillaries 1/16" OD x 1/32 ID, mass balance.

**Materials:** PLGA (50:50 ratio of lactide to glycolide, Mₙ 7 - 17 kD), >99% Acetone, DI H₂O.

**Temperature:** Ambient (20 to 25 °C)

### Method 1: Synthesis of 215 ± 10 nm PLGA seeds

1. Dissolved PLGA (20 g) in acetone (800 mL).
2. Stirred (50 rpm) deionised H₂O (3000 mL) in a 5L beaker using an overhead stirrer equipped with 55 mm PTFE anchor stirrer.
3. Infused PLGA acetone solution into deionised H₂O using the peristaltic pump attached to the 9-port microfluidic manifold with 8x capillaries at a rate of 1 mL/min/capillary. Capillaries were as close to the bottom of the beaker as possible without interfering with PTFE anchor.
4.Upon complete infusion of acetone solution into deionised H₂O, capillaries were removed and acetone allowed to fully evaporate off under continued stirring for ca. 3 days.

### Method 2: Synthesis of 295 ± 10 nm PLGA seeds

1. Dissolved PLGA (12 g) in acetone (300mL).
2. Stirred (50 rpm) deionised H₂O (300 mL) in the 0.5 L ChemGlass jacketed reactor using an overhead stirrer equipped with 55 mm PTFE anchor stirrer
3. Infused PLGA acetone solution into deionised H₂O using the peristaltic pump attached to the 9-port microfluidic manifold with 4x capillaries at a rate of 1 mL/min/capillary. Capillaries were as close to the bottom of the reactor vessel as possible without interfering with PTFE anchor.
4. Acetone allowed to evaporate off until a mean particle diameter of 300 nm is achieved.
5. PLGA suspension diluted with deionised H₂O (200 mL).
6. Remaining acetone allowed to evaporate off for ca. 1 day.
7. PLGA suspension diluted with deionised H₂O to achieve 7 mg/mL.

**Results:** PLGA seed particles are produced as a white suspension, concentration = 6-7 mg/mL, mean hydrodynamic particle diameter by Dynamic Light Scattering (DLS) = 215 ± 10 nm (method 1) or 295 ± 10 nm (method 2), polydispersity index (PDI) by DLS ≤ 0.1.

### Example 2 - Manufacture of the particles

### 2.1 Reaction:

**Process:** Seeded emulsion polymerisation (free radical) on 0.5 L scale.

**Equipment**: 0.5 L ChemGlass jacketed reactor, reactor lid with 4x 24/40 SN ports and 1× 45/50 CN port, 3x 24/40 PTFE stoppers, 1x 45/50 PTFE stirrer shaft support, overhead stirrer with 55 mm PTFE anchor, PTFE temperature probe (PT100 with LEMO connection), Huber MiniStat 230 Pilot ONE, 500 mL measuring cylinder, glass funnel, 40 mL glass vials, spatula, mass balance.

**Materials:** PLGA seeds suspension, ≥98 % HEMA, ≥ 99 % MMA, ≥99 % potassium persulfate (KPS), ≥98 % EGDMA, ≥98 % DEGDMA, DI H₂O.

**Method:** The following synthesis process is a general method which was used to make three different formulations. The specific feed compositions of the three formulations are set out in Table 1.
1. Monomer mixture and KPS solution (2 to 5 mg/mL in deionised H₂O) were prepared.
2. Heat jacketed reactor to process (reaction) temperature using Huber Ministat 230: flow rate was set to 2500 rpm, appropriate maximum-, minimum- and over- temperature limits were selected for the oil bath to ensure the desired process temperature was reached.
3. PLGA seeds and deionised H₂O were charged to achieve 3 g PLGA seeds in a total volume of 500 mL.
4. Process temperature was allowed reach 70.0±0.5 °C
5. When process temperature was 70.0±0.5 °C, the monomer mixture was charged to the reaction vessel and the mixture was stirred.
6. KPS solution was charged.
7. Polymerisation was then quenched at desired time by setting Huber Ministat 230 to 15 °C until a process temperature ≤ 25 °C is reached. Stirring was throughout.

**Table 1 - Feed composition of batch IDs: SGP130, SGP136 and SGP137**

| **Mass (mg)** | **SGP130** | **SGP136** | **SGP137** |
|---|---|---|---|
| PLGA seeds (Mean particle diameter) | 2397 (297 nm) | 3000 (209 nm) | 3000 (297 nm) |
| HEMA | 1562 | 1806 | 1804 |
| MMA | 7552 | 9302 | 9326 |
| EGDMA | 4000 | 7008 | 7005 |
| DEGDMA | 1620 | 0 | 0 |
| KPS^{c} | 35 | 35 | 35 |

**Results:** Crude formulations were produced as white suspension containing unreacted monomers present as clear oily residue/droplets. Mean hydrodynamic particle diameter by DLS = 390±10 nm or 450±10 nm depending on whether the 215 nm or 295 nm PLGA seeds were used respectively. Cup-shaped particles are observed under TEM/SEM imaging.

### 2.2 Washing

**Process:** Water/solvent washing using repeat centrifugation/vortex cycles.

**Equipment:** Thermostatic centrifuge, vortex, 50 mL centrifuge tubes.

**Materials:** Crude formulation from step 2.1, ≥99 % acetonitrile, DI water.

### Method:

1. Crude formulation was transferred into 50 mL centrifuge tubes
2. Centrifuge was then run at 4500 rpm, 20 °C for 30 mins
3. Supernatant was discarded
4. Pellet was resuspended in 10 mL deionised H₂O using vortex
5. 40 mL acetonitrile was added and tubes were shaken well
6. Centrifuge was then run at 4500 rpm, 20 °C for 30 mins
7. Supernatant was discarded
8. Pellet was resuspended in 10 mL deionised H₂O using vortex
9. 40 mL deionised H₂O was added and tubes shaken well
10. Centrifuge was then run at 4500 rpm, 20 °C for 30 mins
11. Supernatant was then discarded
12. Pellet was resuspended in 10 mL deionised H₂O

**Results:** Washed particles were produced as a white suspension. Further characterisation was not performed at this stage.

### 2.3 Drying

**Process:** Oven drying with grinding using a magnetic stirring.

**Equipment:** Fan oven, 70 µm sieve, aluminium pan, mass balance, 26 mm magnetic stirrer bar, stirrer plate.

**Materials**: Washed particles prepared in 2.2.

### Method:

1. Transferred washed suspension (ca. 10 mL) into an aluminium pan through a 70 µm sieve.
2. Dried the suspension (50 °C, 10 hours)
3. Transferred dried particles to 25 mL Duran bottle containing a 26 mm magnetic stirrer bar
4. Capped bottle and gently stirred dried particles to produce a very fine powder (ca. 30 mins)
5. Uncapped bottle and further dried the particles (50 °C, 30 mins)
6. Capped bottle and further ground the particles (15 mins)
7. Uncapped bottle and further dried the particles (50 °C, 30 mins)
8. Capped bottle and allowed particles and bottle to cool fully to ambient temperature (i.e. 20 - 25 °C).

**Results:** Dried formulations were produced as a very fine white powder. Further characterisation was not performed at this stage.

### 2.4 Resuspending

**Process:** Resuspension using magnetic stirring following.

**Equipment:** Stirrer plate, 25 mL Duran bottle, 26 mm magnetic stirrer bar, 36 µm sieve, 100 mm diameter crystallisation dish

**Materials:** Dried particles (prepared in 2.3), deionised water

### Method:

1. Deionised H₂O was added to dried particles to target a final suspension concentration of 30 mg/mL
2. This mixture was stirred gently for 3 hours ensuring that all dried powder stuck to the inner wall of the Duran bottle has been taken into suspension
3. The suspension was filtered through 36 µm sieve into a clean and dry Duran bottle containing a 26 mm magnetic stirrer bar
4. Stirring of the suspension was continued until a PDI by DLS ≤ 0.2 is achieved
5. Concentration was calculated using a thermogravimetric method
6. Suspension was diluted to 20 mg/mL and stored in a capped vessel

**Results:** Resuspended particles were produced as a white suspension at a concentration of 20 mg/mL. Further characterisation is detailed in section 2.5.

### Example 3 - Chemical and Physical characterisation

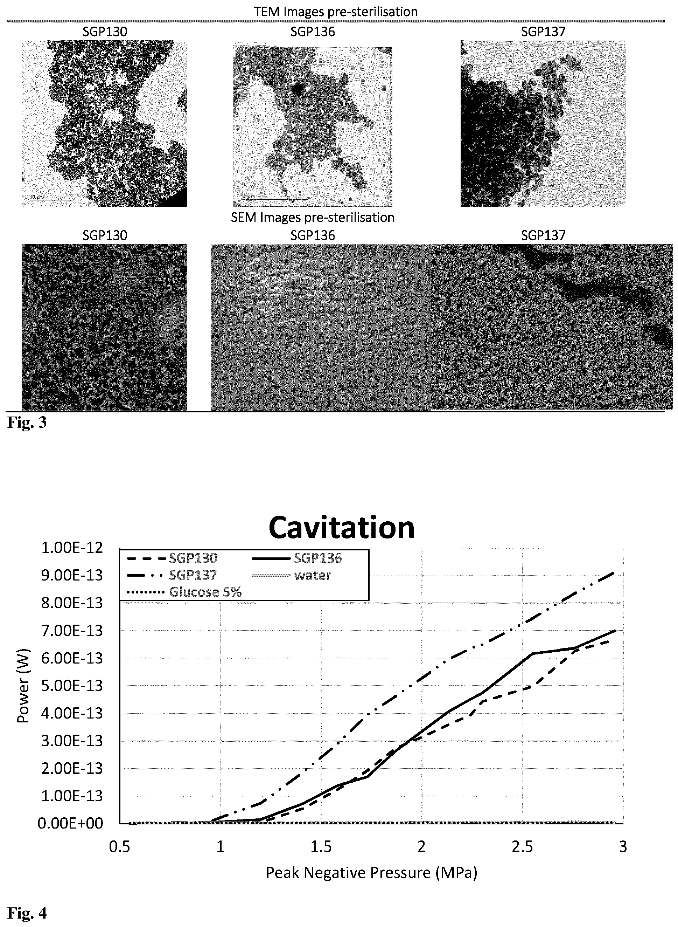

Characterisation of three formulations SGP130, SGP136 and SGP137 pre- and post-sterilisation is given below in Table 2. All characterisation is performed on the resuspended formulations except TEM size analysis which is performed on dried formulations. The particles were further characterised in SEM and TEM images, as shown in Figure 3.

**Table 2 - Characterisation of SGP130, SGP136 and SGP137 pre- and post-sterilisation**

| | | **SGP130** | **SGP136** | **SGP137** |
|---|---|---|---|---|
| **Physical characterisation** | | | | |
| **Mean particle diameter by DLS, D_{H}, 0.1 mg/mL in DI H₂O (n=9)^{a} (nm)** | Pre-sterilisation | 628 ± 24 | 497 ± 41 | 587 ± 8 |
| | Post-sterilisation | 928 ± 17^{c} | 496 ± 10 | 575 ± 8 |
| **PDI, 0.1 mg/mL in DI H₂O (n=9)^{a} (nm)** | Pre-sterilisation | 0.185 ± 0.034 | 0.126 ± 0.056 | 0.121 ± 0.029 |
| | Post-sterilisation | 0.288 ± 0.029 | 0.094 ± 0.044 | 0.102 ± 0.028 |
| **pH, 20 mg/mL (n=3)** | Pre-sterilisation | 6.3 ± 0.1 | 6.7 ± 0.0 | 6.2 ± 0.0 |
| | Post-sterilisation | 4.5 ± 0.0 | 4.6 ± 0.0 | 4.3 ± 0.0 |

| **TEM size analysis^{b}** | | | | |
|---|---|---|---|---|
| **Particle distribution** | Pre-sterilisation | 1.4 % >800 nm | 0.0% >700 nm | 0.8 % >800 nm |
| | | 6.2 % >700 nm | 0.8 % >600 nm | 2.4 % >700 nm |
| | | 13.8 % >600 nm | 4.4 % >500 nm | 6.6 % >600 nm |
| | | 40.6 % >500 nm | 23.2 % >400 nm | 29.8 % >500 nm |
| | | 62.4 % >400 nm | 78.4 % >300 nm | 80.0 % >400 nm |
| | | 66.2 % >300 nm | 99.8 % >200 nm | 99.6 % >300 nm |
| | | 100 % >200 nm | 100 % >100 nm | 100 % >200 nm |
| | | Min = 202 nm | Min = 188 nm | Min = 286 nm |
| | | Max = 1314 nm | Max = 626 nm | Max = 1040 nm |
| | | Mean = 444 nm | Mean = 359 nm | Mean = 471 nm |
| | | SD = 164 nm | SD = 72 nm | SD = 88 nm |
| | | (n=500) | (n=500) | (n=500) |
| | Post-sterilisation | 2.2 % >800 nm | 0.2 % >700 nm | 0.4 % >800 nm |
| | | 3.6 % >700 nm | 0.4 % >600 nm | 1.4 % >700 nm |
| | | 13.2 % >600 nm | 4.2 % >500 nm | 6.4 % >600 nm |
| | | 32.4 % >500 nm | 26.2 % >400 nm | 24.0 % >500 nm |
| | | 64.6 % >400 nm | 68.8 % >300 nm | 74.2 % >400 nm |
| | | 71.6 % >300 nm | 99.8 % >200 nm | 99.4 % >300 nm |
| | | 99.8 % >200 nm | 100 % >100 nm | 100 % >200 nm |
| | | 100 % >100 nm | Min = 196 nm | Min = 278 nm |
| | | Min = 197 nm | Max = 716 nm | Max = 872 nm |
| | | Max = 967 nm | Mean = 351 nm | Mean = 456 nm |
| | | Mean = 435 nm | SD = 80 nm | SD = 83 nm |
| | | SD = 151 nm | (n=500) | (n=500) |
| | | (n=500) | | |
| **Mean cavity diameter (nm)** | Pre-sterilisation | Not measured due to insufficient contrast in image | 197 ± 39 | 296 ± 60 |
| | | | (n=100) | (n=100) |
| | Post-sterilisation | Not measured due to insufficient contrast in image | 220 ± 35 | 280 ± 55 |
| | | | (n=100) | (n=100) |

| | | | | |
|---|---|---|---|---|
| ^{a} 3 repeat sample preparations analysed 3 times per preparation ^{b} TEM images analysed using ImageJ. Cavity diameter could only be measured from side, so the number of particles counted is reduced. ^{c} Increase in size believed to be caused by agglomeration of particles during sterilisation | | | | |

### Example 4 - Cavitation characterisation

*In-vitro* cavitation assessment was carried out using manufacturing testing rig (MTR) described below. For each formulation, the cavitation power is shown in Figure 4.

The Manufacturing Test Rig (MTR) used was designed and developed by OxSonics Ltd. and consists of the following equipment:
- Sonic Concepts Transducer Power Output (TPO) is a combination of a radio-frequency signal generator and power amplifier for generating ultrasound signals to a transducer.
- Sonic Concepts 500kHz focused transducer is an off-the-shelf geometrically focused transducer for transmitting therapeutic ultrasound.
- Sonic Concepts Hydrophone is an off-the-shelf ultrasound receive transducer with large bandwidth for recording ultrasound signals like cavitation emissions.
- Sonic Concept Wattmeter is an off-the-shelf electrical power meter for radiofrequency (RF) pulsed and continuous wave (CW) measurements.
- Allen Avionics 5MHz High Pass Filter is an off-the-shelf passive analog filter of electrical radio-frequency signals.
- SRS Preamplifier is an off-the-shelf four-channel analog signal preamplifier for low noise amplification of electronic signals up to 350MHz.
- Pico Technology Picoscope is an off-the-shelf PC-based, USB-interfaced analog to digital signal acquisition system for recording electronic signals received such as those from an ultrasound receive transducer.
- Lenovo Laptop is a standard off-the-shelf, mid-range Windows-based laptop.
- World Precision Instruments Infusion Pump is an off-the-shelf syringe pump for controlled infusion of liquids.

**Method:** Dilute the particles to pre-determined concentration in µg/mL then pass the diluted solution of particles into the system to measure the cavitation at different pressures (MPa).

### Example 5 - Degradation in-vitro

A degradation study was executed under accelerated conditions to give an estimated real-time degradation profile, determined through the Arrhenius equation. The data suggests that SGP137 is fully degraded after 5 weeks (equivalent to 11 months under real-time conditions at 37 °C), and SGP136 is fully degraded after 6 weeks (14 months under real-time conditions at 37 °C). SGP130 and 20D07G did not fully degrade within the timeframe of the study. *In vivo* degradation profile may be affected by biological factors such as the presence of enzymes accelerating the degradation rate. Such factors were not considered in this *in-vitro* study.

A number of samples were prepared containing particles under oxidative conditions: 3% H₂O₂, pH 4.5-5.0 and maintained at a temperature of 69 ± 1 °C for a period of 6 weeks in an incubator shaker (200 rpm). At 7-day intervals, the samples were removed from the incubator and multiple analytical techniques (i, e. optical density, TEM, particles counting) were used to characterise the samples and assess the rate of degradation.

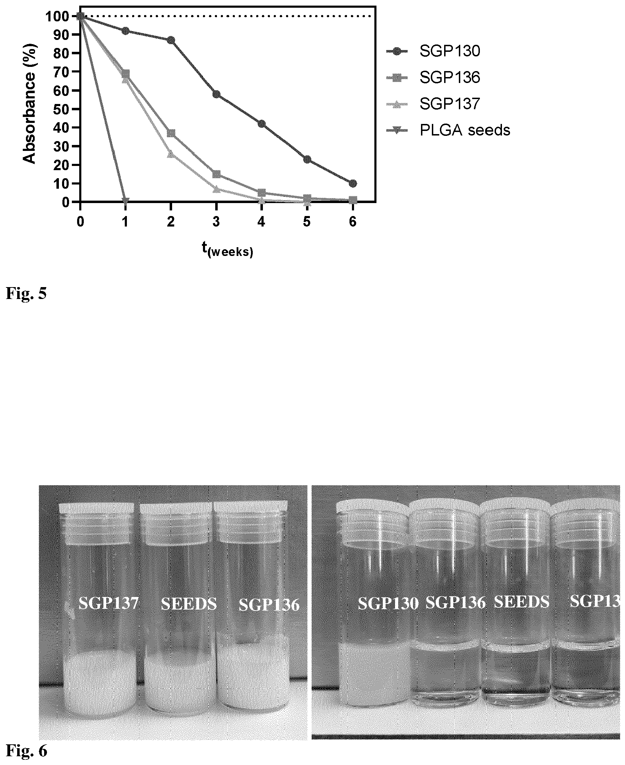

Throughout the degradation study the largest change in any property for the formulations was optical density, the data is presented in Figure 5 and 6. The control samples behaved as expected, the readily degradable PLGA seeds sample following 1 week was a completely transparent solution indicating complete degradation by this timepoint.

Each of the three formulations displayed different behaviour with regards to the changes in optical density. SGP136 and SGP137 exhibited the quickest rate of decrease, with SGP136 having a slightly slower rate than SGP137, although further repeat samples would be needed to confirm whether this is statistically significant. The former reached an optical density of zero by six weeks, whereas the later reach zero optical density by five weeks.

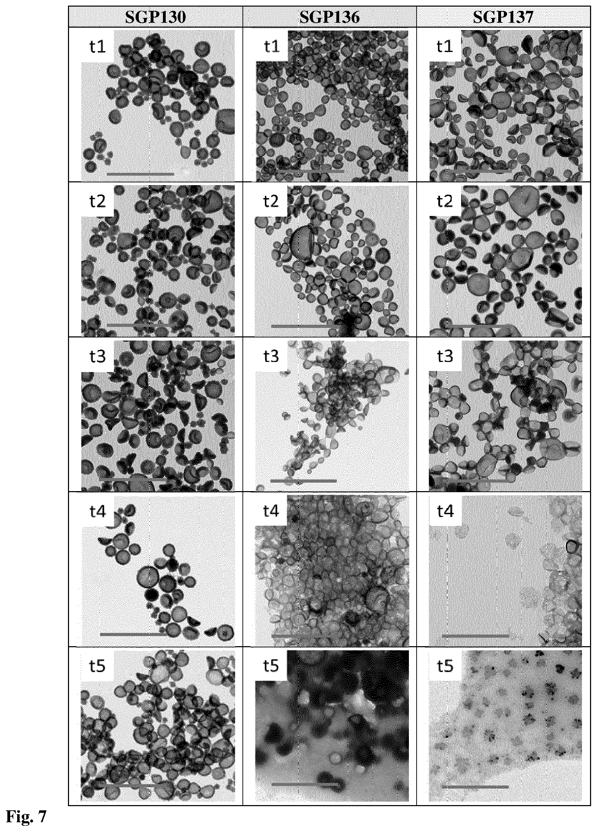

This data correlated well with observations from the TEM imaging (Figure 7). TEM imaging is another analytical technique that was able to discern clear differences between the formulations.

At one week (t1) clear differences in the density of the particles could be observed specifically between the less dense SGP136 and SGP137 and the denser SGP130 and 20D07G (evident by the difference in contrast, between the particles and the background). This could explain the difference in degradation rate between SGP130 and SGP136 and SGP137.

Throughout the degradation study images of the samples were taken each week. Images were not taken at six weeks however as at five weeks there was no evidence of particles in SGP137, the most degradable formulation.

Intact particles and retention of the cup shaped morphology for some particles could also be observed for SGP130 at five weeks. The largest changes observed by TEM were for SGP136 and SGP137, where complete loss of morphology was observed for both samples. For both formulations the particles got successively thinner week by week but clear differences between the formulations could be seen at four weeks. Particles could barely be observed at four weeks for SGP137, instead highly porous fragments of the crosslinked polymer could be seen which subsequently disappeared by five weeks where all that could be observed was salt and residues of break down products (small molecules, oligomers etc). For SGP136 particles were still observed at four weeks and at five weeks, however at five weeks they were barely visible within a significant amount of salt and break down products residues. Much like the optical density data, TEM images demonstrate that SGP137 was the quickest degrading formulation.

Throughout the study, dynamic light scattering was used to assess the mean particle diameter, the PDI and the derived count rate of the particles. The latter is a quantitative measure of the amount of light scattered by the particles, influenced both by particle concentration and particle size. The data is shown in Table 3.

**Table 3 - DLS data for the formulations (SGP130, SGP136 and SGP137).**

| | | **t0** | **t1** | **t2** | **t3** | **t4** | **t5** | **t6** |
|---|---|---|---|---|---|---|---|---|
| **SGP130** | Mean size (nm) | 824 | 3106 | 612 | 497 | 557 | 511 | 616 |
| | PDI | 0.189 | 0.788 | 0.177 | 0.091 | 0.126 | 0.109 | 0.202 |
| | Derived count rate (kcps) | 54812 | 69609 | 26236 | 21642 | 16813 | 8496 | 3322 |
| **SGP136** | Mean size (nm) | 437 | 1218 | 398 | 394 | 391 | 500 | 416 |
| | PDI | 0.061 | 0.518 | 0.066 | 0.058 | 0.083 | 0.311 | 0.245 |
| | Derived count rate (kcps) | 74737 | 60718 | 41742 | 16077 | 4033 | 812 | 176 |
| **SGP137** | Mean size (nm) | 477 | 751 | 479 | 482 | 456 | 254 | * |
| | PDI | 0.088 | 0.194 | 0.050 | 0.049 | 0.059 | 0.383 | |
| | Derived count rate (kcps) | 69615 | 52525 | 22239 | 12456 | 1469 | 64 | |

| | | | | | | | | |
|---|---|---|---|---|---|---|---|---|
| *SGP137 t6 analysis not performed due to the derived count rate being too low at t5. | | | | | | | | |

A small drop in the derived count rate was observed at six weeks, which is consistent with the drop in optical density observed, this could represent degradation of the particles starting to occur. For the formulations no trend in the mean particle size or PDI was observed with consistent values up to six weeks for SGP130 and up to five weeks for SGP136 and SGP137. At this point a significant increase in the PDI was observed for SGP136 and SGP137, however this is only due to the quality of the data being collected. The derived count rate for both samples (812 kcps for SGP136 and 64 kcps for SGP137) is becoming too low as the concentration of particles remaining in the sample continues to decrease. At this point the instrument fails to collect accurate data and hence the PDI becomes broader, making mean particle size values less accurate as well. Although this data cannot be used quantitatively, it demonstrates the differences in the rates at which the particles are degrading and supports the data from the TEM and optical density analyses that shows SGP137 is degrading the quickest out of the formulations. The lack of any significant change in the mean particle size for the formulations up to five weeks is also further evidence for the degradation mechanism being a bulk process.

## Claims

1. A cup-shaped micro- or nanoparticle comprising a first polymer and a second polymer;
wherein the first polymer is a biodegradable polymer;
the second polymer is a crosslinked copolymer of two or more different methacrylate monomers and at least one crosslinking agent, wherein the at least one cross-linking agent comprises one or more dimethacrylates; and
wherein the cup has a cavity having an opening of at least 50 nm in size.

2. A cup-shaped particle according to claim 1, wherein the first polymer is selected from poly(lactic-co-glycolic acid) (PLGA), polyglycolic acid (PGA), poly(L-lactic acid) (PLA), poly(dioxanone), poly(ethylene succinate), poly (butylene succinate), chitosan, and poly(hydroxyalkanoate);
preferably, wherein the first polymer is PLGA which, more preferably, comprises a blend of lactic acid to glycolic acid of from 25:75 to 80:20 weight percent, preferably from 40:60 to 60:40 weight percent.

3. A cup-shaped particle according to any one of claims 1 to 2, wherein the two or more different methacrylate monomers are methyl methacrylate (MMA) and (hydroxyethyl)methacrylate (HEMA);
preferably, wherein the second polymer comprises MMA, crosslinking agent and HEMA in a weight percentage ratio of (30-55):(40-60):(5-10).

4. A cup-shaped particle according to any one of claims 1 to 3, wherein the one or more dimethacrylates are selected from ethylene glycol dimethacrylate (EGDMA) and di(ethylene glycol) dimethacrylate (DEGDMA) and combinations thereof, preferably, wherein the crosslinking agent is EGDMA.

5. A cup-shaped particle according to any one of the preceding claims wherein the weight percentage ratio of the first polymer to the second polymer is (first polymer: second polymer) from 25:75 to 45:55.

6. A cup-shaped particle according to any one of the preceding claims, wherein a gas pocket is present in the cavity.

7. A cup-shaped particle according to any one of the preceding claims, wherein the particle is from 100 nm to 1000 nm in size, preferably from 150 nm to 600 nm in size.

8. A cup-shaped particle according to any one of the preceding claims, wherein the particle comprises one or more release agents;
preferably, wherein the or each release agent is a drug.

9. A method of producing a cup-shaped particle according to any one of the preceding claims, the method comprising:
- providing a seed particle, wherein said seed particle comprises a first polymer, which is a biodegradable polymer; and
- polymerising one or more monomers and/or pre-oligomers and/or pre-polymers and at least one crosslinking agent on the surface of the seed particle to produce a cup having a cavity.

10. A method according to claim 9, wherein the cup-shaped particle comprises one or more release agents, and the method further comprises providing one or more release agents (a) as a coating on the seed particle; and/or (b) during polymerisation; and/or (c) to the exterior surface of the cup.

11. A pharmaceutical composition comprising a plurality of cup-shaped particles as defined in any one of claims 1 to 8, or obtainable according to claim 9 or claim 10, and a pharmaceutically acceptable carrier or diluent which is a liquid medium.

12. A solid composition comprising a plurality of particles as defined in any one of claims 1 to 8, or obtainable according to claim 9 or claim 10, the cup-shaped particles being in dried form.

13. A cup-shaped particle as defined in any one of claims 1 to 8 or obtainable according to claim 9 or claim 10, or a composition according to claim 11 or claim 12, for use in a method of treatment or diagnosis of a human or animal subject, wherein the method comprises administering the cup-shaped particle, or the composition, to the subject and exposing the subject to ultrasound.

14. A cup-shaped particle or composition for use according to claim 13, wherein the method further comprises administering a drug to the subject, and wherein the exposure to ultrasound causes delivery of the drug to a target site;
preferably, wherein said target site is selected from a tumour, a fibrous tissue, an arterial stenosis, and a thrombus.

15. An ultrasound contrast agent comprising a cup-shaped particle as defined in any one of claims 1 to 8 or obtainable according to claim 9 or claim 10, or a composition according to claim 11 or claim 12.
